# EUROPEAN PATENT APPLICATION

(11) **EP 4 484 364 A1**
(43) Date of publication of application: **01.01.2025**
(21) Application number: 23182234.7
(22) Date of filing: 28.06.2023
(51) Int. Cl.: C01B 3/38, C01B 3/50, C10G 2/00, C25B 1/04, C07C 1/12, C07C 9/04

(54) **INTEGRATED PROCESS FOR METHANE PRODUCTION AND DRY METHANE REFORMING**

(71) Applicant: BP P.L.C., London SW1Y 4PD (GB)
(72) Inventor: The designation of the inventor has not yet been filed
(74) Representative: Hamer, Christopher K.

(57) **Abstract**

The present disclosure relates generally to integrated processes for the production of methane and its use in dry methane reforming. In one aspect, the present disclosure provides process for producing a stream containing hydrogen and carbon monoxide, the process comprising: providing a methane synthesis feed stream comprising hydrogen and carbon dioxide; contacting the methane synthesis feed stream with a methane synthesis catalyst (e.g., in a methane synthesis reactor) to form a methane synthesis product stream comprising methane and water; providing a dry methane reformation feed stream comprising carbon dioxide and at least a portion of the methane of the methane synthesis product stream; contacting the dry methane reformation feed stream with a dry methane reformation catalyst (e.g., in a dry methane reformation reactor) to produce a dry methane reformer product stream comprising carbon monoxide and hydrogen.

## Description

### BACKGROUND OF THE DISCLOSURE

### FIELD

The present disclosure relates to integrated processes for the production of methane and also dry methane reforming.

### TECHNICAL BACKGROUND

The conversion of synthesis gas into hydrocarbons by the Fischer-Tropsch process has been known for many years. The growing importance of alternative energy sources has resulted in renewed interest in the Fischer-Tropsch (FT) process as it allows an alternative route to high-quality fuels and feedstock chemicals through use of bio-derived carbon sources.

FT processes are typically used to produce linear hydrocarbons, which can be used in the production of fuels, as well as oxygenates which can also be useful in the production of fuels and otherwise serve as valuable feedstock chemicals.

A variety of transition metals have been identified to be catalytically active in the conversion of synthesis gas into hydrocarbons and oxygenated derivatives thereof. In particular, cobalt, nickel, and iron have been studied, often in combination with a support material, of which the most common are alumina, silica and carbon.

Typically, Fischer-Tropsch reactions utilize carbon monoxide as the carbon source due to its increased reactivity compared to carbon dioxide. But utilization of carbon dioxide is of great interest due to its prevalence as a waste gas and low cost, and it would be desirable to use carbon dioxide as an ultimate feedstock to a Fischer-Tropsch process.

Accordingly, there remains a need to develop processes to more efficiently utilize carbon dioxide in the production of hydrocarbons.

### SUMMARY

The present inventors have developed integrated processes for the production of methane and its use in dry methane reforming to provide an H₂/CO stream comprising hydrogen and carbon monoxide.

Thus, in one aspect, the disclosure provides a process for producing an H₂/CO stream comprising hydrogen and carbon monoxide, the process comprising:
providing a methane synthesis feed stream comprising hydrogen and carbon dioxide;
contacting the methane synthesis feed stream with a methane synthesis catalyst (e.g., in a methane synthesis reactor) to form a methane synthesis product stream comprising methane and water;
providing a dry methane reformation feed stream comprising carbon dioxide and at least a portion of the methane of the methane synthesis product stream;
contacting the dry methane reformation feed stream with a dry methane reformation catalyst (e.g., in a dry methane reformation reactor) to produce a dry methane reformer product stream comprising carbon monoxide and hydrogen.
At least a portion of the dry methane reformer product can be provided to the H₂/CO stream.

In another aspect, the present disclosure provides a process for the production of hydrocarbons, the process comprising
providing an H₂/CO stream comprising hydrogen and carbon monoxide according to the methods as described herein; and
providing a Fischer-Tropsch feed stream comprising the H₂/CO stream;
contacting (e.g., in a Fischer-Tropsch reactor) the Fischer-Tropsch feed stream with a Fischer-Tropsch catalyst to produce a Fischer-Tropsch product stream comprising hydrocarbons, water and optionally oxygenated hydrocarbons.

Other aspects of the disclosure will be apparent to those skilled in the art in view of the description that follows.

### BRIEF DESCRIPTION OF THE DRAWINGS

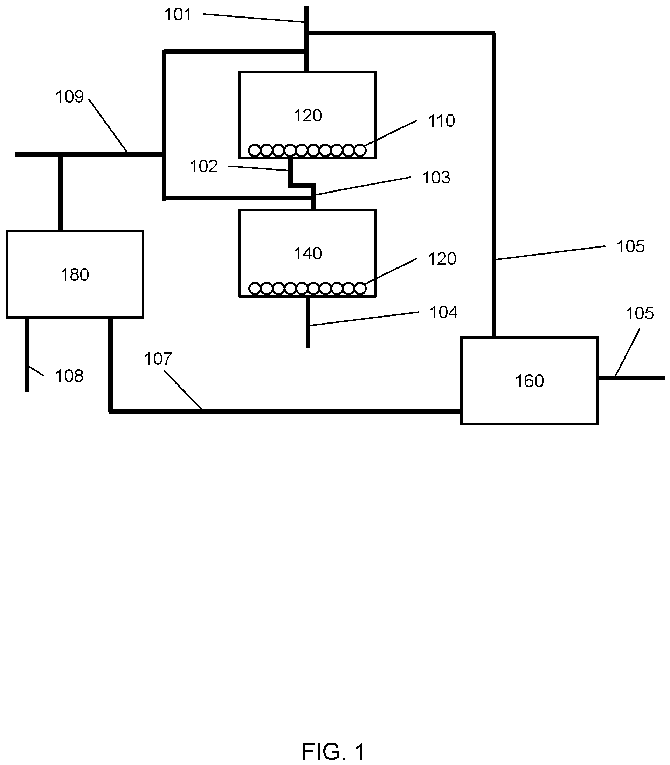

FIG. 1 provides a process schematic according to one embodiment of the disclosure.

### DETAILED DESCRIPTION

The inventors have noted that one possible way to use carbon dioxide in a Fischer-Tropsch process is through the so-called "reverse water gas shift reaction," in which carbon dioxide is reacted with hydrogen to produce carbon monoxide and water. The produced carbon monoxide may then, together with hydrogen, be subjected to a Fisher-Tropsch synthesis. However, this conversion conventionally requires exceptionally high temperatures, often in excess of 900 °C, and thus may be energetically unfavorable.

The present inventors have devised an alternative scheme for the use of carbon dioxide in hydrocarbon synthesis. The present inventors have noted that carbon dioxide and hydrogen can be reacted to substantially form methane (i.e., instead of to substantially form carbon monoxide as in the reverse water-gas shift), The present inventors have noted that methane, in turn, can be converted to carbon monoxide by reaction with carbon dioxide in a dry methane reforming process. These processes can be performed at relatively low temperatures, and thus can provide significant advantages over processes operating through the intermediary of a reverse water-gas shift.

Thus, in one aspect, the disclosure provides a process for producing an H₂/CO stream comprising hydrogen and carbon monoxide, the process comprising:
providing a methane synthesis feed stream comprising hydrogen and carbon dioxide;
contacting the methane synthesis feed stream with a methane synthesis catalyst (e.g., in a methane synthesis reactor) to form a methane synthesis product stream comprising methane and water;
providing a dry methane reformation feed stream comprising carbon dioxide and at least a portion of the methane of the methane synthesis product stream;
contacting the dry methane reformation feed stream with a dry methane reformation catalyst (e.g., in a dry methane reformation reactor) to produce a dry methane reformer product stream comprising carbon monoxide and hydrogen.
At least a portion of the dry methane reformer product can be provided to the H₂/CO stream.

In another aspect, the present disclosure provides for processes for the production of hydrocarbons, the process comprising
providing an H₂/CO stream comprising hydrogen and carbon monoxide according to the methods as otherwise described herein; and
providing a Fischer-Tropsch feed stream comprising the H₂/CO stream;
contacting (e.g., in a Fischer-Tropsch reactor) the Fischer-Tropsch feed stream with a Fischer-Tropsch catalyst to produce a Fischer-Tropsch product stream comprising hydrocarbons, water and optionally oxygenated hydrocarbons.

Advantageously, the process as otherwise described herein may use as a feed hydrogen gas prepared through electrolysis. For example, in various embodiments as otherwise described herein, the process as otherwise described herein further comprises electrolyzing water to form a hydrogen gas-containing stream, and providing at least a portion of the hydrogen gas-containing stream to the methane synthesis feed stream. Electrolysis of water to generate hydrogen may be powered by electricity from a renewable source of energy. Accordingly, in various embodiments, the electrolysis is performed using electricity from a renewable source of energy, such as solar power (including photovoltaic solar, or solar thermal power), hydroelectric power, wind power, and geothermal power. Numerous methods of electrolysis are known in the art. For example, electrolysis may be performed on water to produce hydrogen gas and oxygen gas. Water electrolysis is described further in U.S. Patent No. 4,312,720, U.S. Patent No. 4,021,323, and U.S. Patent No. 4,094,751, each of which is incorporated by reference in their entirety.

Hydrogen may be categorized based on the process for producing it. Accordingly, in various embodiments as otherwise described herein, at least a portion of the hydrogen (e.g., at least 50%, at least 75%, at least 90% or at least 95%) of the methane synthesis feed stream is one or more of grey hydrogen (produced conventionally from natural gas, or methane using steam reformation without capturing associated greenhouse gases), brown or black hydrogen (hydrogen produced using black coal or lignite), green hydrogen (made using water electrolysis powered by electricity from renewable sources of energy), pink hydrogen (made from electrolysis powered by nuclear energy), turquoise hydrogen (made from methane pyrolysis to produce hydrogen and solid carbon), blue hydrogen (made from steam reforming of natural gas, where the resulting carbon dioxide is captured), yellow hydrogen (made from electrolysis using solar power) and orange hydrogen (made from electrolysis using wind power). In particular embodiments, at least a portion of the hydrogen of the methane synthesis feed stream is one or more of green hydrogen, pink hydrogen, or blue hydrogen (e.g., green hydrogen).

Carbon dioxide is a common waste material, and often desirable to be removed from waste streams rather than be vented to the atmosphere. Such capture of carbon dioxide is critical to the implementation of many initiatives as it serves to lower the carbon footprint of the associated process. Advantageously, the carbon dioxide utilized in the processes described herein may be carbon dioxide collected from the atmosphere or that would otherwise have been released into the atmosphere, e.g., from a combustion or other industrial process. The carbon dioxide may be captured, where it is collected or absorbed after release from an industrial process, or harvested directly from the atmosphere. Methods of carbon dioxide capture are known to those of skill in the art. In various embodiments, at least a portion of the CO₂ of the methane synthesis feed stream is from direct air capture. Additionally or alternatively, carbon dioxide is often scrubbed from industrial effluent, especially processes that generate large amounts of carbon dioxide as a byproduct.

In various embodiments as otherwise described herein, the carbon dioxide of the methane synthesis feed stream and/or the dry methane reformation feed stream comprises captured carbon dioxide and/or carbon dioxide from biomass gasification, e.g., comprises at least 50%, at least 75%, at least 90%, or at least 95% captured carbon dioxide and/or carbon dioxide from biomass gasification.

As noted above, biomass can be used to provide CO₂ to the claimed processes. One source of biomass is agricultural products in the form of dedicated energy crops such as switchgrass, miscanthus, bamboo, sorghum, tall fescue, kochia, wheatgrass, poplar, willow, silver maple, eastern cottonwood, green ash, black walnut, sweetgum, and sycamore. Another biomass source is agricultural waste or agricultural crop residue. Conventional agricultural activities, including the production of food, feed, fiber, and forest products, generate large amounts of waste plant material. Examples of such materials include corn stover, wheat straw, oat straw, barley straw, sorghum stubble, and rice straw. A third biomass source is forestry residues left after timber operations. Biomass may also be in the form of commercial waste, industrial waste, sewage sludge, and municipal waste, which includes commercial and residential garbage, including yard trimmings, paper and paperboard, plastics, rubber, leather, textiles, and food waste. Accordingly, in various embodiments as otherwise described herein, the at least a portion of the CO₂ of the methane synthesis feed stream is derived from a biomass source. For example, in particular embodiments, at least a portion (e.g., at least 20%, at least 50%, at least 75%, at least 90%, or at least 95%) of the CO₂ is derived from biomass gasification, for example, agricultural biomass or municipal waste biomass. Additional sources of agricultural biomass will be apparent to one of skill in the art as dictated by local availability, economics, and process compatibility.

To generate carbon dioxide from a carbon-containing material, such as biomass, the material is typically subjected to gasification. Gasification involves heating the material under controlled conditions to generate gaseous streams of carbon monoxide, hydrogen, and carbon dioxide. Controlled amounts of other reactants, such as oxygen and/or steam, may be used to tune the process. Gasification conditions are tuned in accordance with the carbon-containing material being gasified in order to efficiently produce gaseous products. The biomass may be any source as described above, or from multiple sources may be combined.

As described herein, methane synthesis may be accomplished through reacting carbon dioxide with hydrogen according to the idealized equation:

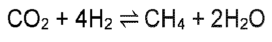

Advantageously, methane synthesis according to the present disclosure can be accomplished without carbon monoxide in the feedstock, as carbon monoxide is a valuable starting material for use in other processes. Accordingly, in various embodiments as otherwise described herein, the methane synthesis feed comprises no more than 5 vol% carbon monoxide, e.g., no more than 3 vol%, or 2 vol%, or 1 vol%, or 0.1 vol% carbon monoxide. In particular embodiments, the methane synthesis feed comprises essentially no carbon monoxide.

In various embodiments as otherwise described herein, the methane synthesis feed stream comprises hydrogen gas and carbon dioxide in a molar ratio in the range of from 0.5:1 to 8:1, e.g., from 1:1 to 6:1, or 2:1 to 5:1.

Optionally, the methane synthesis feed stream may also comprise one or more additional gases. In such embodiments, the methane synthesis feed stream may further comprise one or more of CO, CH₄, and N₂. In particular embodiments, the methane synthesis feed stream comprises an carrier gas, wherein the carrier gas includes one or more of CH₄ or N₂, e.g., N₂. In various embodiments as otherwise described herein, at least 50 vol% of the of the methane synthesis mixture is made up of the combination of hydrogen, carbon dioxide and nitrogen, e.g., in a total amount of at least 60 vol%, at least 70 vol%, at least 80 vol%, or at least 90 vol%.

The methane synthesis catalyst and/or Fischer-Tropsch synthesis catalyst may be any suitable catalyst; many such catalysts are known in the art. For example, the methane synthesis catalyst and/or Fischer-Tropsch synthesis catalyst may be an iron-based catalyst or a cobalt-based catalyst. Other examples of suitable catalysts include those based on nickel or platinum. A person of ordinary skill in the art may select a suitable catalyst in light of the present disclosure.

As described above, in some embodiments the methane synthesis catalyst (e.g., a supported methane synthesis catalyst) and/or Fischer-Tropsch synthesis catalyst (e.g., supported Fischer-Tropsch synthesis catalyst) used in the processes of the disclosure may comprise in the range of 1 wt% to 35 wt% iron or cobalt on an elemental basis. Notably, many catalysts that are conventionally used in Fischer-Tropsch processes are surprisingly suitable for the methane synthesis processes described herein. The person of ordinary skill in the art will, based on the disclosure herein, select a suitable amount of iron or cobalt. For example, in various embodiments, the methane synthesis catalyst and/or Fischer-Tropsch synthesis catalyst comprises iron or cobalt in an amount in the range of 1-30 wt%, or 1-25 wt%, or 1-20 wt%, or 2-35 wt%, or 2-30 wt%, or 2-25 wt%, or 2-20 wt%, or 5-35 wt%, or 5-30 wt%, or 5-25 wt%, on an elemental basis. In various particular embodiments, the methane synthesis catalyst and/or Fischer-Tropsch synthesis catalyst comprises cobalt in an amount in the range of 2-20 wt%, e.g., 2-15 wt%, or 2-10 wt%, or 5-20 wt%, or 5-15 wt%, or 5-10 wt%, or 7-20 wt%, or 7-15 wt%, on an elemental basis.

In various embodiments, the methane synthesis catalyst includes iron as a catalytic metal. In various alternative embodiments, the methane synthesis catalyst includes cobalt as a catalytic metal.

In various embodiments, the Fischer-Tropsch synthesis catalyst includes iron as a catalytic metal. In various alternative embodiments, the Fischer-Tropsch synthesis catalyst includes cobalt as a catalytic metal.

In various embodiments, the suitable methane synthesis catalysts and/or Fischer-Tropsch synthesis catalysts may also include a wide variety of other transition metals. For example, a variety of promoters, such as one or more of manganese, ruthenium, palladium, platinum, rhodium, rhenium, chromium, nickel, iron, molybdenum, tungsten, zirconium, gallium, thorium, lanthanum, cerium, copper and mixtures thereof may be included. Promoter is typically used in a catalytic metal (e.g., iron or cobalt) to promoter atomic ratio of up to 250:1, e.g., up to 125:1, or up to 25:1, or up to 10:1. In various such embodiments, the one or more promoters are present in the methane synthesis catalyst and/or Fischer-Tropsch synthesis catalyst in an amount from 0.5 wt% to 15 wt%, e.g., 0.5 wt% to 10 wt%, or 1 wt% to 3 wt%, on an elemental basis, based on the total weight of the supported synthesis catalyst. In particular embodiments, the promoter is manganese. In other embodiments, the methane synthesis catalyst and/or Fischer-Tropsch synthesis catalyst does not contain any such promoters.

In various embodiments as otherwise described herein, the methane synthesis catalyst and/or Fischer-Tropsch synthesis catalyst may further comprise a support material (e.g., the methane synthesis catalyst may be a supported methane synthesis catalyst; and/or the Fischer-Tropsch synthesis catalyst may be a supported Fischer-Tropsch synthesis catalyst). The support material serves to bind and structurally support the catalyst particles and may also influence the catalytic activity and product selectivity. In various embodiments as otherwise described herein, the support material includes at least one of alumina, zirconia, titania, silica, zinc oxide, ceria, or combinations thereof. In particular embodiments, the support material comprises one of alumina, zirconia, zinc oxide, ceria, silica and titania, for example the support material is one of alumina, zirconia, zinc oxide, ceria, silica and titania. In yet other particular embodiments, the support material comprises titania (e.g., at least 90 wt% titania, or at least 95 wt% titania, or at least 99 wt% titania), for example the support material is titania.

The methane synthesis catalyst and/or Fischer-Tropsch synthesis catalyst used in accordance with the present disclosure may be prepared by any suitable method. Preferably, the methane synthesis catalyst and/or Fischer-Tropsch synthesis catalyst may be prepared by a process in which the cobalt is impregnated on to the support material.

A suitable impregnation method, for example, comprises impregnating a support material with metal-containing compound, which is thermally decomposable to the oxide form. Impregnation of the support material with the metal-containing compound may be achieved by any suitable method of which the skilled person is aware, for instance by vacuum impregnation, incipient wetness or immersion in excess liquid.

The incipient wetness technique is so-called because it typically requires that the volume of impregnating solution be predetermined so as to provide the minimum volume of solution necessary to just wet the entire surface of the support, with substantially no excess liquid. The excess solution technique as the name implies, requires an excess of the impregnating solution, the solvent being thereafter removed, usually by evaporation.

In various embodiments, the support material, if present, may be in the form of a powder, granulate, shaped particle, such as a preformed sphere or microsphere, or extrudate. Reference herein to a powder or granulate of a support material is understood to refer to free flowing particles of a support material or particles of support material that have undergone granulation and/or sieving to be a particular shape (e.g. spherical) and size range. Reference herein to an "extrudate" is intended to mean a support material that has undergone an extrusion step and therefore may be shaped.

It will be understood that the support material may be in any form provided it is suitable for use as a support for a methane synthesis catalyst and/or Fischer-Tropsch synthesis catalyst. Preferred support materials are substantially free of extraneous components that might adversely affect the catalytic activity of the system. Thus, in various embodiments support materials are at least 95 wt% pure, more preferably at least 98 wt% pure and most preferably at least 99 wt% pure. Impurities preferably amount to less than 1 wt%, more preferably less than 0.5 wt% and most preferably less than 0.25 % w/w. The pore volume of the support is preferably more than 0.15 mL/g and preferably more than 0.30 mL/g. The average pore radius (prior to impregnation) of the support material is 10 to 500 Å, preferably 15 to 100 Å, more preferably 20 to 80 A and most preferably 25 to 60 Å. The BET surface area is suitably from 2 to 1000 m²/g, preferably from 10 to 600 m²/g, more preferably from 15 to 300 m²/g, and most preferably 30 to 150 m²/g.

In various embodiments as otherwise described herein, particularly in the case of fixed catalyst bed reactor systems, it may be beneficial to perform methane synthesis and/or Fischer-Tropsch catalysis with a shaped catalyst, such as an extrudate. For instance, it is known that, for a given shape of catalyst particles, a reduction in the size of the catalyst particles in a fixed bed gives rise to a corresponding increase in pressure drop through the bed. Thus, the relatively large shaped particles cause less of a pressure drop through the catalyst bed in the reactor compared to the corresponding powdered or granulated supported catalyst. Shaped particles, such as extrudates, also generally have greater strength and experience less attrition, which is of particular value in fixed bed arrangements where bulk crush strength is desirably high.

The solvent of the impregnating solution(s) may be either an aqueous solvent or a non-aqueous, organic solvent. Suitable non-aqueous organic solvents include, for example, alcohols (e.g. methanol, ethanol and/or propanol), ketones (e.g. acetone), liquid paraffinic hydrocarbons and ethers. Alternatively, aqueous organic solvents, for example an aqueous alcoholic solvent, may be employed. Preferably, the solvent of the impregnating solution(s) is an aqueous solvent.

It will be appreciated that where the support material is in powder or granulate form, once impregnated with suitable metal compounds, such as a cobalt containing compound, iron-containing compound, or the like, the impregnated support material may be extruded or formed into shaped particles at any suitable stage before or after drying and calcining.

Impregnation of the support material is usually followed by drying of the impregnating solution in order to effect precipitation of the metal-containing compound on to the support material and preferably also to remove bound solvent of the impregnating solution (e.g. water). As will be appreciated, in embodiments where an extrusion is performed, complete drying and removal of solvent (e.g. bound solvent) of the impregnating solution may occur after forming of a shaped particle, for example by extrusion. Drying is suitably conducted at temperatures from 50 °C to 150 °C, preferably 75 °C to 125 °C. Suitable drying times are, for example, from 5 minutes to 72 hours. Drying may suitably be conducted in a drying oven or in a box furnace, for example, under the flow of an inert gas at elevated temperature.

Preparation of the methane synthesis catalyst and/or Fischer-Tropsch synthesis catalyst may involve a calcination step. As will be understood, calcination is required for converting any metal-containing compound which has been impregnated on the support material into an oxide of the metal. For example, wherein the metal is cobalt, calcination leads to thermal decomposition of the cobalt-containing compound, and not merely removal of bound solvent of an impregnating solution, as for instance in the case of drying.

Calcination may be performed by any method known to those of skill in the art, for instance in a fluidized bed or rotary kiln at a temperature of at least 250 °C, preferably from 275 °C to 500 °C.

The methane synthesis catalyst and/or Fischer-Tropsch synthesis catalyst may conveniently be subjected to reductive activation by any known means of which the skilled person is aware. For example, in embodiments wherein the catalyst comprises cobalt, activation conditions are such that are capable of converting cobalt oxide to the active cobalt metal (e.g., to form a reduced methane synthesis catalyst and/or reduced Fischer-Tropsch synthesis catalyst), such as through the use of a reducing gas. In particular embodiments, the reducing gas comprises hydrogen gas. The step of forming a reduced synthesis catalyst may be carried out batch wise or continuously in a fixed bed, fluidised bed or slurry phase reactor, or in-situ in the same reactor as will be subsequently used for the methane synthesis reaction. Reduction is suitably performed at a temperature of from 150 °C to 350 °C, e.g., from 150 °C to 325 °C, or from 200 °C to 325 °C.

In various embodiments as otherwise described herein, the process comprises electrolyzing water to produce a hydrogen gas containing stream. In various such embodiments, the process may further comprise activating the methane synthesis catalyst and/or Fischer-Tropsch catalyst by contacting it with at least a portion of the hydrogen gas containing stream produced by the electrolyzing step.

In some embodiments, the methane synthesis catalyst and/or Fischer-Tropsch synthesis catalyst may be passivated in order to prevent deactivation upon exposure to air. Passivation may be desirable in order to store, transport, load and/or unload the catalyst. Once installed in the reactor, the catalyst may be re-activated. Accordingly, in various embodiments as otherwise described herein, the process further comprises passivating the methane synthesis catalyst and/or Fischer-Tropsch synthesis catalyst by contacting the methane synthesis catalyst and/or Fischer-Tropsch synthesis catalyst with a passivation agent (e.g., a passivating agent comprising oxygen) to form a passivated methane synthesis catalyst and/or Fischer-Tropsch synthesis catalyst; and re-activating the methane synthesis catalyst and/or Fischer-Tropsch synthesis catalyst by contacting the methane synthesis catalyst and/or Fischer-Tropsch synthesis catalyst with a reducing agent at temperature of no more than 350 °C. In particular embodiments, the passivation agent is oxygen, optionally admixed with one or more of water, nitrogen, and carbon dioxide. In various embodiments as otherwise described herein, the process further comprises, prior to the re-activating step, transporting the passivated methane synthesis catalyst and/or Fischer-Tropsch synthesis catalyst and charging a reactor bed with the passivated methane synthesis catalyst and/or Fischer-Tropsch synthesis catalyst.

As will be appreciated, in some embodiments the gaseous reactant mixture supplied to the Fischer-Tropsch synthesis catalyst to form a reduced Fischer-Tropsch synthesis catalyst in situ, without requiring any preceding or distinct reductive activation step.

The person of ordinary skill in the art will perform the processes described herein using any desirable reaction systems. For example, a wide variety of reactors can be used, e.g., a fixed bed reactor, a slurry bed reactor, or a fluid bed reactor.

Advantageously, the processes of the present disclosure may be conducted at relatively low temperatures compared to other routes for the conversion of carbon dioxide into hydrocarbons, such as through the reverse water gas shift reaction. In various embodiments as otherwise described herein, the contacting of the methane synthesis feed stream with the methane synthesis catalyst is performed at a temperature in the range of 150 °C to 325 °C (e.g., in the range of 150 °C to 300 °C, or 150 °C to 275 °C, or 150 °C to 270 °C, or 150 °C to 260 °C, or 150 °C to 250 °C, or 175 °C to 325 °C, or 175 °C to 275 °C, or 175 °C to 270 °C, or 175 °C to 260 °C, or 175 °C to 250 °C, or 200 °C to 325 °C, or 200 °C to 275 °C, or 200 °C to 270 °C, or 200 °C to 260 °C, or 200 °C to 250 °C). In various embodiments as otherwise described herein, the contacting of the methane synthesis feed stream with the methane synthesis catalyst is performed at a pressure in the range from 10 to 100 barg (from 1 to 10 MPa). As the person of ordinary skill in the art will appreciate, "barg" is a unit of gauge pressure, being the pressure in bars above ambient or atmospheric pressure. For example, in various embodiments, the contacting is performed at a pressure in the range of 20 barg to 80 barg, e.g., in the range of 20 barg to 60 barg, or 20 barg to 50 barg, or 20 barg to 40 barg.

As used herein, "selectivity" for a given component is measured as the molar fraction of a particular reactant that is reacted in the process (i.e., not including any unreacted portion of that particular reactant) and is converted to that product. For example, in the reaction of carbon dioxide and hydrogen to provide product components including methane, "selectivity" for a given component is defined as the molar fraction of carbon dioxide that is reacted in the process and is converted to the product of interest, not including any unreacted carbon dioxide.

Advantageously, the methane synthesis processes described herein can produce a product composition with a high selectivity for methane. In various embodiments as otherwise described herein, the selectivity for methane is at least 80% (e.g., at least 85%, or at least 90%, or at least 95%). It may be desirable to limit the selectivity for C₅₊ hydrocarbons. In various embodiments as otherwise described herein, the carbon dioxide is reacted with a C₅₊ selectivity of no more than 10%, e.g., no more than 8%, or no more than 7%, or no more than 5%, or no more than 4%, or no more than 3%. In various embodiments as otherwise described herein, the carbon dioxide is reacted with a C₂₊ selectivity of no more than 25%, e.g., no more than 20%, or no more than 15%, or no more than 10%, or no more than 5%. It may also be desirable to limit the selectivity for oxygenates. Oxygenates are oxygen-containing molecules, such as alcohols, ethers, esters, carboxylic acids, and the like (but not including carbon dioxide or carbon monoxide). In various embodiments as otherwise described herein, the carbon dioxide is reacted with an oxygenate selectivity of no more than 10%, e.g., no more than 8%, or no more than 7%, or no more than 5%, or no more than 4%, or no more than 3%.

The methane synthesis processes of the present disclosure advantageously efficiently convert carbon dioxide to methane. High conversion of carbon dioxide is a major challenge in the art, as carbon dioxide can often be relatively inert except for in extreme temperature and/or pressure regimes. The present inventors have identified methods that allow high conversion of carbon dioxide in relatively mild conditions. Accordingly, in various embodiments as otherwise described herein, the methane synthesis product stream is provided with a carbon dioxide conversion of at least 25%, e.g., at least 30% or at least 35%, or at least 40%, or at least 45%, or at least 50%. As used herein, "conversion" of carbon dioxide is the molar fraction of carbon dioxide that is converted to other species in the reaction. Of course, the person of ordinary skill in the art will appreciate that any unreacted carbon dioxide can be separated and recycled for use as part of the carbon dioxide feed.

As noted above, one conventional method of conversion of carbon dioxide is the reverse water gas shift reaction, in which carbon dioxide and hydrogen are converted to carbon monoxide and water: 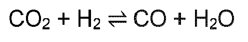 However, to drive product conversion the reverse water gas shift reaction is typically performed at high reaction temperatures, often in excess of 900 °C. Thus, reverse water gas shift is costly from an energy standpoint, and specialized equipment must be used. Further, the process consumes hydrogen which is often expensive in terms of process economics. An advantage of the processes of the present disclosure is the ability to convert carbon dioxide to useful higher hydrocarbons, without using the reverse water gas shift reaction. Accordingly, in various embodiments as otherwise described herein, the process does not include a reverse water gas shift reaction. It is possible that a small proportion of carbon dioxide is converted to carbon monoxide by the reverse water-gas shift reaction as a reaction side product during normal operation of the processes described herein. Accordingly, the absence of a reverse water-gas shift reaction is understood to mean that there is not a distinct reaction zone dedicated to the reverse water-gas shift reaction. Desirably, the methane synthesis processes described herein have a selectivity for carbon monoxide of less than 1%, e.g., less than 1000 ppm or even less than 100 ppm.

In other embodiments as otherwise described herein, the process includes a water gas shift reaction, however, in particular embodiments, the water gas shift reaction is operated so as to consume carbon monoxide and water and generate carbon dioxide and hydrogen.

Methane is a valuable feedstock for a variety of chemical processes and when made using carbon dioxide derived from sources which may generally be considered as renewable sources (e.g., capture from atmosphere or other chemical or industrial processes, or biomass conversion); such methane can be considered as a carrier for such carbon. Similarly, when made using green hydrogen and/or blue hydrogen as described herein (e.g., hydrogen formed from water electrolysis or steam reforming), the methane can be considered as a carrier of hydrogen from such sources.

Methane reforming can be used to convert methane to carbon monoxide and hydrogen, and the present inventors have noted that this can be especially useful in Fischer-Tropsch synthesis. Several reforming techniques are known in the art, typically utilizing the reaction of methane with water and/or oxygen. Examples include steam reforming, autothermal reforming, gas heated reforming, and partial oxidation reforming.

However, various processes of the present disclosure advantageously do not rely chiefly on methane reforming of the above types, wherein oxygen and/or water is used as a primary reactant. Rather, in various embodiments as otherwise described herein, the processes use dry methane reforming. Dry methane reforming reacts carbon dioxide with methane to form hydrogen and carbon monoxide according to the equation:

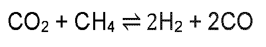

Accordingly, in various embodiments as otherwise described herein, the process comprises providing a dry methane reformation feed stream comprising carbon dioxide methane. In various embodiments, at least a portion of the methane of the dry methane reformation feed stream is comprised of at least a portion of the methane synthesis product stream. As described herein, various embodiments of the methane synthesis can produce water as an additional product. Accordingly, it can be advantageous to reduce the water content of the methane synthesis product stream before the associated methane is subjected to dry methane reforming. Accordingly, in various embodiments as otherwise described herein, provision of at least a portion of the methane of the methane synthesis product stream to the dry methane reformation feed stream comprises separating at least a portion of the water from the methane of the methane synthesis product stream. For example, in particular embodiments, the separation provides a methane stream having no more than 10% of a water content of the methane synthesis product stream (e.g., no more than 7.5%, or no more than 5%, or no more than 3%, or no more than 1%).

The separated water may be recycled to other applications. For example, in various embodiments as otherwise described herein, the process comprises electrolyzing at least a portion of the separated water to form a hydrogen gas containing stream, and providing at least a portion of the hydrogen gas containing stream to the methane synthesis feed stream, the process further comprising providing at least a portion of the separated water as a feed to the electrolyzing.

As described herein, the dry methane reformation feed stream advantageously contains low water content. For example, in various embodiments as otherwise described herein, the dry methane reformation feed stream comprises no more than 10 vol% water, e.g., no more than 7.5 vol% water, or no more than 5 vol% water, or no more than 3 vol% water, or no more than 1 vol% water).

As described herein, methane synthesis may be carried out to react carbon dioxide with hydrogen to form methane and water. In such reactions, in various instances a portion of the carbon dioxide does not react but is carried into the methane synthesis product stream. The present inventors note that CO₂ is also a reactant in the dry methane reforming reaction. Accordingly, in various embodiments as otherwise described herein, the methane synthesis product stream comprises carbon dioxide, and at least a portion of the carbon dioxide of the methane synthesis product stream is provided to the dry methane reformation feed. Additionally or alternatively, in particular embodiments, at least a portion of the carbon dioxide of the methane synthesis product stream is recycled to the methane synthesis feed stream.

Dry methane reforming is described for example, in International Patent Application Publications nos. 2014/195904 and 2021/247829, each of which is hereby incorporated herein in its entirety.

In various embodiments as otherwise described herein, the dry methane reformation feed stream has a methane:carbon dioxide molar ratio in the range of from 1:4 to 8:1. Any suitable catalyst may be used as known in the art. For example, in various embodiments as otherwise described herein, the dry methane reforming catalyst comprises a transition metal or oxide thereof, optionally disposed on a refractory oxide support. In particular embodiments, the transition metal or oxide thereof comprises at least one of nickel, cobalt, ruthenium, rhodium, and iron. In some particular embodiments, the transition metal or oxide thereof comprises nickel.

In various embodiments as otherwise described herein, the dry methane reformation product stream is provided with a methane conversion of at least 15%, e.g., at least 20% or at least 25%, or at least 30%, or at least 45%, or at least 50%. As used herein, "conversion" of methane is the molar fraction of methane that is converted to other species in the dry methane dry methane reformation process. Of course, the person of ordinary skill in the art will appreciate that any unreacted methane can be separated and recycled for use as part of the feed to the dry methane dry methane reformation process.

As described herein, dry methane reformation ideally results in products containing 1:1 molar ratios of hydrogen gas and carbon monoxide. However, this ratio may be not be ideal for downstream processes. For example, Fischer-Tropsch hydrocarbon synthesis (e.g., for the formation of C₅₊ hydrocarbons) typically requires a molar ratio of hydrogen to carbon monoxide of greater than 1:1. Accordingly, in various embodiments as otherwise described herein, the process further comprises adjusting the methane reformer product stream to provide an H₂/CO stream containing hydrogen and carbon monoxide, with a H₂:CO ratio in the range of 1.5:1 to 3:1.

The adjusting may be accomplished through a variety of means, including combinations thereof. For example, in various embodiments, the adjusting comprises subjecting at least a portion of the dry methane reformer product stream to the water-gas shift reaction (e.g., in a water-gas shift reactor). In various embodiments, the adjusting comprises adding hydrogen gas to the methane reformer product stream. For example, wherein the process comprises electrolyzing water to produce a hydrogen gas containing stream (e.g., as otherwise described herein), and using at least a portion of the hydrogen gas containing stream in the adjusting step. In various embodiments, the adjusting comprises removing carbon monoxide from the methane reformer product stream.

As described herein, as aspect of the present disclosure is the production of an H₂/CO stream containing hydrogen and carbon monoxide. As described above, in various embodiments, the production of the H₂/CO proceeds through the production of methane from carbon dioxide, followed by the dry reforming of methane to produce carbon monoxide and hydrogen. Accordingly, the stream containing hydrogen and carbon monoxide may further include additional gases. For example, in various embodiments as otherwise described herein, the H₂/CO stream comprises methane in an amount in the range of 0.1 wt% to 50 wt% (e.g., 1 wt% to 50 wt%, 5 wt% to 50 wt%, 10 wt% to 50 wt%, 15 wt% to 50 wt%, 20 wt% to 50 wt%, 25 wt% to 50 wt%, 0.1 wt% to 40 wt%, 0.1 wt% to 30 wt%, 0.1 wt% to 25 wt%, 0.1 wt% to 20 wt%, 1 wt% to 30 wt%, 2 wt% to 30 wt%, 5 wt% to 30 wt%). For example, in various embodiments as otherwise described herein, the H₂/CO stream comprises carbon dioxide in an amount in the range of 0.1 wt% to 50 wt% (e.g., 1 wt% to 50 wt%, 5 wt% to 50 wt%, 10 wt% to 50 wt%, 15 wt% to 50 wt%, 20 wt% to 50 wt%, 25 wt% to 50 wt%, 0.1 wt% to 40 wt%, 0.1 wt% to 30 wt%, 0.1 wt% to 25 wt%, 0.1 wt% to 20 wt%, 1 wt% to 30 wt%, 2 wt% to 30 wt%, 5 wt% to 30 wt%).

And as noted herein, mixtures of carbon monoxide and hydrogen can be useful in the synthesis of higher hydrocarbons via the Fischer-Tropsch process. Accordingly, another aspect of the disclosure is a process for the production of hydrocarbons, the processes comprising:
providing a H₂/CO stream comprising hydrogen and carbon monoxide according to the process as otherwise described herein;
providing a Fischer-Tropsch feed stream comprising at least a portion of the H₂/CO stream;
contacting the Fischer-Tropsch feed stream with a Fischer-Tropsch catalyst (e.g., in a Fischer-Tropsch reactor) to produce a Fischer-Tropsch product stream comprising C₅₊ hydrocarbons.

The product stream can be provided, for example, with a selectivity for C₅₊ hydrocarbons of at least 50% and/or a selectivity for oxygenates of at least 20%. In various embodiments as otherwise described herein, the Fischer-Tropsch hydrocarbon catalyst used in this Fischer-Tropsch process may have a composition similar to that of the methane synthesis catalyst as otherwise generically described herein. For example, the Fischer-Tropsch catalyst may be the same as the methane synthesis catalyst utilized for the production of methane. The person of skill in the art may choose appropriate Fischer-Tropsch hydrocarbon synthesis parameters in light of the present disclosure and in view of the existing state of the art. Suitable techniques for Fischer-Tropsch hydrocarbon synthesis, especially with regard to the synthesis of C₅₊ hydrocarbons and/or oxygenates, are described, for example, in International Patent Application Publication No. 2019/154885, hereby incorporated by reference herein in its entirety. But the person of ordinary skill in the art can adapt other Fischer-Tropsch processes to use the H₂/CO streams described herein.

Accordingly, in various embodiments, the Fischer-Tropsch catalyst is a cobalt-containing supported Fischer-Tropsch catalyst. For example, in various embodiments, the Fischer-Tropsch feed stream has a H₂:CO ratio in the range of 1:1 to 3:1, and the Fischer-Tropsch catalyst contains at least 1 wt% cobalt and at least 0.5 wt% manganese.

The Fischer-Tropsch process as otherwise described herein may also have an advantageously high C₅₊ selectivity. For example, in various embodiments, the process has a C₅₊ selectivity of at least 75%, for example, at least 80%, or at least 85%, or at least 90%. The Fischer-Tropsch process as otherwise described herein may also have an advantageously low methane selectivity. For example, in various embodiments, the process has a methane selectivity of no more than 6%, for example, no more than 5%, or no more than 4%, or no more than 3%.

CO conversion is defined as moles of CO reacted to other species/moles of CO fed x 100. C₅₊ hydrocarbon selectivity is defined as moles of CO attributed to C₅₊ hydrocarbons (i.e., moles of C in C5+ hydrocarbons)/moles of CO converted x 100. Alcohol selectivity is defined as moles of CO attributed to alcohols (i.e., moles of C in alcohols)/moles of CO converted x 100.

Conventional Fischer-Tropsch temperatures may be used in order to prepare liquid hydrocarbons in accordance with the present disclosure, using the catalysts described herein. For example, the temperature of the contacting of a mixture of hydrogen and the gaseous carbon oxide (e.g., in the form of a synthesis gas mixture) with a Fischer-Tropsch catalyst may suitably be in the range from 100 to 400 °C, such as from 100 to 350 °C, or 100 to 300 °C, or 100 to 250 °C, or 150 to 400 °C, or 150 to 350 °C, or 150 to 300 °C. In various embodiments, the contacting is conducted at a temperature of no more than 350 °C, e.g., no more than 325 °C, or no more than 300 °C, or no more than 280 °C. The pressure of the contacting (i.e., the temperature of the Fischer-Tropsch reaction) can in various embodiments suitably be in the range from 10 to 100 bara (from 1 to 10 MPa), such as from 15 to 75 bara (from 1.5 to 7.5 MPa), or from 20 to 50 bara (from 2.0 to 5.0 MPa). For example, in various embodiments the contacting is conducted at a pressure of no more than 7.5 MPa absolute.

In particular embodiments, the temperature of the Fischer-Tropsch reaction is in the range from 150 to 350 °C, more preferably from 180 to 300 °C. In preferred embodiments, the pressure of the Fischer-Tropsch reaction is in the range from 10 to 100 bar (from 1 to 10 MPa), more preferably from 10 to 60 bar (from 1 to 6 MPa) and most preferably from 20 to 45 bar (from 2 to 4.5 MPa).

In addition to hydrocarbons, the Fischer-Tropsch product stream may further include other components from the feed stream and/or formed *in situ,* such as light hydrocarbons (e.g., methane and/or C₂-C₄ hydrocarbons), nitrogen, water, and/or carbon dioxide. Advantageously, certain species may be separated and recycled to other processes. For example, in various embodiments as otherwise described herein, the Fischer-Tropsch product stream comprises carbon dioxide, and the process further comprises providing at least a portion of the carbon dioxide of the Fischer-Tropsch product stream to the methane synthesis feed stream. In various embodiments as otherwise described herein, wherein the Fischer-Tropsch product stream comprises carbon dioxide, and wherein the process further comprises providing at least a portion of the carbon dioxide of the Fischer-Tropsch product stream to the dry methane reformation feed stream. In various embodiments as otherwise described herein, wherein the Fischer-Tropsch product stream comprises methane, and wherein the process further comprises providing at least a portion of the methane of the Fischer-Tropsch product stream to the methane synthesis feed stream. In various embodiments as otherwise described herein, wherein the Fischer-Tropsch product stream comprises methane, and wherein the process further comprises providing at least a portion of the methane of the Fischer-Tropsch product stream to the dry methane reformation feed stream.

The Fischer-Tropsch product stream will often include water. In various embodiments as otherwise described herein, the process comprises electrolyzing at least a portion of the water of the Fischer-Tropsch to form a hydrogen gas containing stream. In various such embodiments, the process further comprises providing at least a portion of the hydrogen gas containing stream to the methane synthesis feed stream. Additionally or alternatively, the process can further include providing at least a portion of the hydrogen gas containing stream to an adjustment of the H₂/CO ratio of the methane reformer product stream as described above.

In various embodiments, the Fischer-Tropsch product stream further comprises light hydrocarbons (e.g., C₁-C₄ hydrocarbons), and the process further comprises separating a light hydrocarbon stream from the Fischer-Tropsch product stream. In particular embodiments, the process further comprises providing at least a portion of the light hydrocarbon stream to a partial oxidation reactor, and reacting the light hydrocarbon stream with oxygen in the partial oxidation reactor to produce a partial oxidation product stream comprising carbon monoxide and hydrogen. In some embodiments, the process further comprises providing at least a portion of the partial oxidation product stream to the H₂/CO stream or to the Fischer-Tropsch feed stream.

As otherwise described herein, in various embodiments the process comprises electrolyzing water (from any of a variety of sources and using electricity from any of a variety of sources as described above) to produce a hydrogen gas containing stream and an oxygen gas containing stream. In various such embodiments, the process further comprises providing at least a portion of such an oxygen gas containing stream to the partial oxidation reactor.

In various embodiments, the partial oxidation reactor may also produce carbon dioxide. Accordingly, in various embodiments, wherein the partial oxidation product stream comprises carbon dioxide, the process further comprises providing at least a portion of the carbon dioxide of the partial oxidation product stream to the methane synthesis feed stream. Additionally or alternatively, wherein the partial oxidation product stream comprises carbon dioxide, the process further comprises providing at least a portion of the carbon dioxide of the partial oxidation product stream to the dry methane reformation feed stream.

Advantageously, the partial oxidation as otherwise described herein can generate net energy. Accordingly, in various embodiments as otherwise described herein, wherein the partial oxidation produces energy, the process further comprises directing the energy to at least one of the methane synthesis, dry methane reformation, Fischer-Tropsch synthesis, a separation (e.g., a separation as otherwise described herein), an electrolysis reaction (e.g., the electrolyzing of water as otherwise described herein), or a boiler to generate steam.

Unless otherwise stated, temperatures referred to in particular embodiments described herein are applied temperatures and not measured catalyst/bed temperatures. Unless otherwise stated, pressures referred to particular embodiments described herein are absolute pressures.

One embodiment of the disclosure is described with respect to the illustrative example of FIG. 1. Methane synthesis feed stream 101 is introduced into methane synthesis reactor 120 containing methane synthesis catalyst 110. The produced methane synthesis product stream 102 is withdrawn from the reactor and fed into dry methane reformation feed stream 103, which enters dry methane reformer 140 containing dry methane reformation catalyst 120. The resulting dry methane reformer product stream 104 is withdrawn. Water feed stream 105 enters electrolysis reactor 160. Hydrogen stream 105 is withdrawn from the electrolysis reactor and introduced into methane synthesis feed stream 101. Oxygen stream 107 is withdrawn from the electrolysis reactor and introduced into partial oxidation reactor 180 along with light hydrocarbon stream 108. Carbon dioxide stream 109 is withdrawn from the partial oxidation reactor and introduced into methane synthesis feed 101 and/or dry methane reformation feed 103.

In various embodiment described above, methane synthesis may be used to provide methane for subsequent dry methane reforming to form an H₂/CO stream, which can then be used in a variety of processes like Fischer-Tropsch synthesis. However, the present inventors have noted that methane may additionally or alternatively be sourced from biogenic sources, such as biogas. Biogas refers to gas produced by the breakdown of organic matter, such as biomass sources including agricultural waste, manure, municipal waste, sewage, and food waste. Typically, crude biogas contains a mixture of carbon dioxide, carbon monoxide, and methane, although post-capture processing may be used to alter the gaseous composition (e.g., through removing carbon monoxide). Biogas is a source of biogenic carbon dioxide, as well as a source of biomethane. The present inventors have noted that biogas can be supply methane and carbon dioxide to a methane reformation process. Accordingly, another aspect of the disclosure is a process for producing a H₂/CO stream containing hydrogen and carbon monoxide, the processing comprising:
providing a dry methane reformation feed stream comprising biogenic carbon dioxide and biomethane;
contacting the dry methane reformation stream with a dry methane reformation catalyst to produce a dry methane reformer product stream comprising carbon monoxide and hydrogen; and
optionally adjusting the methane reformer product stream to provide a stream containing hydrogen and carbon monoxide with a H₂:CO ratio in the range of 1.5:1 to 3:1.

For example, in various embodiments as otherwise described herein, the dry methane reformation feed stream comprises captured biogas (e.g., at least one of the biogenic carbon dioxide and biomethane is derived from biogas).

In various embodiments, the dry methane reformation stream may comprise a mixture of captured biogas and at least a portion of a methane synthesis product stream as otherwise described herein. Accordingly, in various embodiments the methane synthesis processes described above can be used to provide additional methane to the dry methane reformation feed stream. Similarly, carbon dioxide from sources as described above can be used to provide additional carbon dioxide to the dry methane reformation feed stream.

In another aspect, the present disclosure provides for a process for the production of hydrocarbons, the process comprising
providing a H₂/CO stream containing hydrogen and carbon monoxide produced by a process as otherwise described herein (e.g., a process comprising contacting a dry methane reformation stream comprising biogenic carbon dioxide and biomethane with a dry methane reformation catalyst);
providing a Fischer-Tropsch feed stream comprising the H₂/CO stream; and
contacting (e.g., in a Fischer-Tropsch reactor) the Fischer-Tropsch feed stream with a Fischer-Tropsch catalyst to produce a Fischer-Tropsch product stream comprising hydrocarbons, water and optionally oxygenated hydrocarbons.
The Fischer-Tropsch portion of this process can be performed, e.g., as otherwise described herein.

Various aspects and embodiments of the disclosure are provided by the following enumerated embodiments, which may be combined in any number and in any fashion not logically or technically inconsistent.

Embodiment 1. A process for producing an H₂/CO stream comprising hydrogen and carbon monoxide, the process comprising:
providing a methane synthesis feed stream comprising hydrogen and carbon dioxide;
contacting the methane synthesis feed stream with a methane synthesis catalyst (e.g., in a methane synthesis reactor) to form a methane synthesis product stream comprising methane and water;
providing a dry methane reformation feed stream comprising carbon dioxide and at least a portion of the methane of the methane synthesis product stream;
contacting the dry methane reformation feed stream with a dry methane reformation catalyst (e.g., in a dry methane reformation reactor) to produce a dry methane reformer product stream comprising hydrogen and carbon monoxide.

Embodiment 2. The process of embodiment 1, further comprising electrolyzing water to form a hydrogen gas containing stream, and providing at least a portion of the hydrogen gas containing stream to the methane synthesis feed stream.

Embodiment 3. The process of embodiment 2, wherein the electrolyzing is performed using electricity from a renewable source.

Embodiment 4. The process of any of embodiments 1-3, wherein at least a portion of the hydrogen of the methane synthesis feed stream is one or more of grey hydrogen, brown hydrogen, green hydrogen, pink hydrogen, turquoise hydrogen, blue hydrogen, yellow hydrogen and orange hydrogen.

Embodiment 5. The process of any of embodiments 1-4, wherein the carbon dioxide of the methane synthesis feed stream comprises captured carbon dioxide and/or carbon dioxide from biomass gasification.

Embodiment 6. The process of any of embodiments 1-5, wherein the carbon dioxide of the methane synthesis feed stream comprises at least 50%, at least 75%, at least 90%, or at least 95% captured carbon dioxide and/or carbon dioxide from biomass gasification

Embodiment 7. The process of any of embodiments 1-6, wherein the methane synthesis feed stream comprises no more than 5 vol% carbon monoxide, e.g., no more than 3 vol%, or 2 vol%, or 1 vol%, or 0.1 vol% carbon monoxide.

Embodiment 8. The process of any of embodiments 1-7, wherein the methane synthesis feed stream comprises hydrogen gas and carbon dioxide in a molar ratio in the range of from .5:1 to 8:1, e.g., from 1:1 to 6:1, or 2:1 to 5:1.

Embodiment 9. The process of any of embodiments 1-8, wherein at least 50 vol% of the of the methane synthesis mixture is hydrogen, carbon dioxide and nitrogen, e.g., in a total amount of at least 60 vol%, at least 70 vol%, at least 80 vol%, or at least 90 vol%.

Embodiment 10. The process of any of embodiments 1-9, wherein the methane synthesis catalyst comprises cobalt.

Embodiment 11. The process of any of embodiments 1-10, wherein the methane synthesis catalyst comprises cobalt in an amount in the range of 1 wt% to 35 wt%, .e.g., in the range of 1-30 wt%, or 1-25 wt%, or 1-20 wt%, or 2-35 wt%, or 2-30 wt%, or 2-25 wt%, or 2-20 wt%, or 5-35 wt%, or 5-30 wt%, or 5-25 wt%, on an elemental basis an elemental basis.

Embodiment 12. The process of any of embodiments 1-9, wherein the methane synthesis catalyst comprises iron.

Embodiment 13. The process of any of embodiments 1-9, wherein the methane synthesis catalyst comprises iron in an amount in the range of 1 wt% to 35 wt%, .e.g., in the range of 1-30 wt%, or 1-25 wt%, or 1-20 wt%, or 2-35 wt%, or 2-30 wt%, or 2-25 wt%, or 2-20 wt%, or 5-35 wt%, or 5-30 wt%, or 5-25 wt%, on an elemental basis an elemental basis.

Embodiment 14. The process of any of embodiments 1-13, wherein the methane synthesis catalyst comprises manganese in the range of 0.5 wt% to 15 wt% on an elemental basis.

Embodiment 15. The process of any of embodiments 1-14, wherein the methane synthesis catalyst is a supported catalyst, wherein the support material comprises at least one of alumina, zirconia, titania, silica, zinc oxide, or ceria.

Embodiment 16. The process of any of embodiments 1-15, wherein the contacting the methane synthesis feed stream with a methane synthesis catalyst is performed at a temperature in the range of 150 °C to 325 °C, e.g., (e.g., in the range of 150 °C to 300 °C, or 150 °C to 275 °C, or 150 °C to 270 °C, or 150 °C to 260 °C, or 150 °C to 250 °C, or 175 °C to 325 °C, or 175 °C to 275 °C, or 175 °C to 270 °C, or 175 °C to 260 °C, or 175 °C to 250 °C, or 200 °C to 325 °C, or 200 °C to 275 °C, or 200 °C to 270 °C, or 200 °C to 260 °C, or 200 °C to 250 °C).

Embodiment 17. The process of any of embodiments 1-16, wherein the contacting the methane synthesis feed stream with a methane synthesis catalyst is performed at a pressure in a range of from 10 to 100 barg (e.g., 20 barg to 80 barg, e.g., in the range of 20 barg to 60 barg, or 20 barg to 50 barg, or 20 barg to 40 barg).

Embodiment 18. The process of any of embodiments 1-17, wherein the contacting the methane synthesis feed stream with a methane synthesis catalyst is performed with a selectivity for methane of at least 80% (e.g., at least 85%, or at least 90%, or at least 95%).

Embodiment 19. The process of any of embodiments 1-18, wherein the contacting the methane synthesis feed stream with a methane synthesis catalyst is performed with a C₅₊ hydrocarbon selectivity of no more than 10%, e.g., no more than 8%, or no more than 7%, or no more than 5%, or no more than 4%, or no more than 3%.

Embodiment 20. The process of any of embodiments 1-19, wherein the contacting the methane synthesis feed stream with a methane synthesis catalyst is performed with a C₂₊ selectivity of no more than 25%, e.g., no more than 20%, or no more than 15%, or no more than 10%, or no more than 5%.

Embodiment 21. The process of any of embodiments 1-20, wherein the contacting the methane synthesis feed stream with a methane synthesis catalyst is performed with an oxygenate selectivity of no more than 10%, e.g., no more than 8%, or no more than 7%, or no more than 5%, or no more than 4%, or no more than 3%.

Embodiment 22. The process of any of embodiments 1-21, wherein the contacting the methane synthesis feed stream with a methane synthesis catalyst is performed with a carbon dioxide conversion of at least 25%, e.g., at least 30%, or at least 35%, or at least 40%, or at least 45%, or at least 50%.

Embodiment 23. The process of any of embodiments 1-22 wherein provision of at least a portion of the methane of the methane synthesis product stream to the dry methane reformation feed stream comprises separating at least a portion of the water from the methane of the methane synthesis product stream.

Embodiment 24. The process of embodiment 23, wherein the separation provides a methane stream having no more than 10% of a water content of the methane synthesis product stream (e.g., no more than 7.5%, or no more than 5%, or no more than 3%, or no more than 1%).

Embodiment 25. The process of embodiment 23 or embodiment 24, wherein the process comprises electrolyzing at least a portion of the separated water to form a hydrogen gas containing stream and an oxygen gas containing stream, and providing at least a portion of the hydrogen gas to the methane synthesis feed stream, the process further comprising providing at least a portion of the separated water as a feed to the electrolyzing.

Embodiment 26. The process of any of embodiments 1-25, wherein the dry methane reformation feed stream comprises no more than 10 vol% water, e.g., no more than 7.5 vol% water, or no more than 5 vol% water, or no more than 3 vol% water, or no more than 1 vol% water).

Embodiment 27. The process of any of embodiments 1-26, wherein the carbon dioxide of dry methane reformation feed stream comprises captured carbon dioxide and/or carbon dioxide from biomass gasification.

Embodiment 28. The process of any of embodiments 1-27, wherein the methane synthesis product stream comprises carbon dioxide, and wherein at least a portion of the carbon dioxide of the methane synthesis product stream is provided to the dry methane reformation feed.

Embodiment 29. The process of any of embodiments 1-28, wherein the dry methane reformation feed stream has a methane:carbon dioxide molar ratio in the range of from 1:4 to 8:1.

Embodiment 30. The process of any of embodiments 1-29, wherein the dry methane reforming catalyst comprises a transition metal or oxide thereof (e.g., at least one of nickel, cobalt, ruthenium, rhodium, and iron), optionally disposed on a refractory oxide support.

Embodiment 31. The process of any of embodiments 1-30, wherein the dry methane reforming has a methane conversion of at least 15%, e.g., at least 20% or at least 25%, or at least 30%, or at least 45%, or at least 50%.

Embodiment 32. The process of any of embodiments 1-31, wherein the process further comprises providing at least a portion of the dry methane reformer product stream to the H₂/CO stream (e.g., providing the dry methane reformer product stream as the H₂/CO stream). Embodiment 33. The process of any of embodiments 1-32, wherein the process further comprises adjusting the methane reformer product stream to provide an H₂/CO stream containing hydrogen and carbon monoxide with a H₂:CO ratio in the range of 1.5:1 to 3:1.

Embodiment 34. The process of embodiment 33, wherein the adjusting comprises subjecting at least a portion of the dry methane reformer product stream to a water-gas shift reaction (e.g., in a water-gas shift reactor).

Embodiment 35. The process of embodiment 33 or embodiment 34, wherein the adjusting comprises adding hydrogen gas to the methane reformer product stream.

Embodiment 36. The process of any of embodiments 33-35, wherein the process comprises electrolyzing water to produce a hydrogen gas containing stream, and using at least a portion of the hydrogen gas containing stream in the adjusting step.

Embodiment 37. The process of any of embodiments 1-36, wherein the H₂/CO stream comprises methane in an amount in the range of 0.1 wt% to 50 wt%.

Embodiment 38. The process of any of embodiments 1-37, wherein the H₂/CO stream comprises carbon dioxide in an amount in the range of 0.1 wt% to 50 wt%.

Embodiment 39. A process for the production of hydrocarbons, the process comprising
providing an H₂/CO stream containing hydrogen and carbon monoxide according to the process of any of embodiments 1-38;
providing a Fischer-Tropsch feed stream comprising the stream containing hydrogen and carbon monoxide;
contacting the Fischer-Tropsch feed stream with a Fischer-Tropsch catalyst (e.g., in a Fischer-Tropsch reactor) to produce a Fischer-Tropsch product stream comprising C₅₊ hydrocarbons.

Embodiment 40. The process of embodiment 39, wherein the Fischer-Tropsch feed stream has a H₂:CO ratio in the range of 1:1 to 3:1.

Embodiment 41. The process of embodiment 39 or embodiment 40, wherein the Fischer-Tropsch catalyst is a cobalt-containing supported Fischer-Tropsch catalyst.

Embodiment 42. The process of embodiment 41, wherein the Fischer-Tropsch catalyst comprises titania as a support.

Embodiment 43. The process of any of embodiments 39-42 , wherein the Fischer-Tropsch catalyst comprises cobalt in an amount of at least 1 wt%, e.g., in the range of 1 wt% to 20 wt% on an elemental basis

Embodiment 44. The process of any of embodiments 39-43, wherein the Fischer-Tropsch catalyst comprises manganese in an amount of at least 0.5 wt%, e.g., in the range of 0.5 wt% to 10 wt% on an elemental basis.

Embodiment 45. The process of any of embodiments 39-44, wherein the Fischer-Tropsch product stream comprises carbon dioxide, and wherein the process further comprises providing at least a portion of the carbon dioxide of the Fischer-Tropsch product stream to the methane synthesis feed stream.

Embodiment 46. The process of any of embodiments 39-45, wherein the Fischer-Tropsch product stream comprises carbon dioxide, and wherein the process further comprises providing at least a portion of the carbon dioxide of the Fischer-Tropsch product stream to the dry methane reformation feed stream.

Embodiment 47. The process of any of embodiments 39-46, wherein the Fischer-Tropsch product stream comprises methane, and wherein the process further comprises providing at least a portion of the methane of the Fischer-Tropsch product stream to the methane synthesis feed stream.

Embodiment 48. The process of any of embodiments 39-47, wherein the Fischer-Tropsch product stream comprises methane, and wherein the process further comprises providing at least a portion of the methane of the Fischer-Tropsch product stream to the dry methane reformation feed stream.

Embodiment 49. The process of any of embodiments 39-48, wherein the Fischer-Tropsch product stream comprises water, and wherein the process comprises electrolyzing at least a portion of the water of the Fischer Tropsch product stream to form a hydrogen gas containing stream.

Embodiment 50. The process of embodiment 49, further comprising providing at least a portion of the hydrogen gas containing stream to the methane synthesis feed stream.

Embodiment 51. The process of embodiment 49 or embodiment 50, further comprising providing at least a portion of the hydrogen gas containing stream to an adjustment of the H₂/CO ratio of the methane reformer product stream.

Embodiment 52. The process of any of embodiments 39-51, further comprising separating a light hydrocarbon stream from the Fischer-Tropsch product stream.

Embodiment 53. The process of embodiment 52, further comprising providing at least a portion of the light hydrocarbon stream to a partial oxidation reactor, and reacting the light hydrocarbon stream with oxygen in the partial oxidation reactor to produce a partial oxidation product stream comprising carbon monoxide and hydrogen.

Embodiment 54. The process of embodiment 53, further comprising providing at least a portion of the partial oxidation product stream to the H₂/CO stream or to the Fischer-Tropsch feed stream.

Embodiment 55. The process of embodiment 53 or embodiment 54, wherein the process comprises electrolyzing water to produce a hydrogen gas containing stream and an oxygen gas containing stream, wherein at least a portion of the oxygen gas containing stream is provided to the partial oxidation reactor.

Embodiment 56. The process of any of embodiments 53-55, wherein the partial oxidation product stream comprises carbon dioxide, wherein the process further comprises providing at least a portion of the carbon dioxide of the partial oxidation product stream to the methane synthesis feed stream.

Embodiment 57. The process of any of embodiments 53-56, wherein the partial oxidation product stream comprises carbon dioxide, wherein the process further comprises providing at least a portion of the carbon dioxide of the partial oxidation product stream to the dry methane reformation feed stream.

Embodiment 58. The process of any of embodiments 53-57, wherein the partial oxidation produces energy, the process further comprising directing the energy to at least one of the methane synthesis, dry methane reformation, Fischer-Tropsch synthesis, a separation process, or an electrolysis process.

Embodiment 59. The process of any of embodiments 53-58, wherein the process comprises electrolyzing water to produce a hydrogen gas containing stream, the process further comprising activating the Fischer-Tropsch catalyst by contacting it with at least a portion of the hydrogen gas containing stream.

Embodiment 60. A process for producing an H₂/CO stream containing hydrogen and carbon monoxide, the processing comprising:
providing a dry methane reformation feed stream comprising biogenic carbon dioxide and biomethane;
contacting the dry methane reformation feed stream with a dry methane reformation catalyst to produce a dry methane reformer product stream comprising carbon monoxide and hydrogen; and
optionally adjusting the methane reformer product stream to provide a stream containing hydrogen and carbon monoxide with a H₂:CO ratio in the range of 1.5:1 to 3:1.

Embodiment 61. The process of embodiment 60, wherein the carbon dioxide and methane of the dry methane reformation feed stream comprise biogas.

Embodiment 62. The process of embodiment 60 or embodiment 61, wherein the dry methane reformation feed stream includes captured carbon dioxide.

Embodiment 63. The process of any of embodiments 60-62, wherein the dry methane reformation is performed as further described in any of embodiments 29-32.

Embodiment 64. The process of any of embodiments 60-63, wherein providing the H2/CO stream further comprises 33-38.

Embodiment 65. A process for the production of hydrocarbons, the process comprising
providing a H₂/CO stream containing hydrogen and carbon monoxide according to the method of any of embodiments 60-64; and
providing a Fischer-Tropsch feed stream comprising the stream containing hydrogen and carbon monoxide;
contacting (e.g., in a Fischer-Tropsch reactor) the Fischer-Tropsch feed stream with a Fischer-Tropsch catalyst to produce a Fischer-Tropsch product stream comprising hydrocarbons, water and optionally oxygenated hydrocarbons.

Embodiment 66. The process of embodiment 65, wherein the Fischer-Tropsch process is performed as described in any of embodiments 40-59.

The particulars shown herein are by way of example and for purposes of illustrative discussion of various embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for producing an H₂/CO stream comprising hydrogen and carbon monoxide, the process comprising:
providing a methane synthesis feed stream comprising hydrogen and carbon dioxide;
contacting the methane synthesis feed stream with a methane synthesis catalyst (e.g., in a methane synthesis reactor) to form a methane synthesis product stream comprising methane and water;
providing a dry methane reformation feed stream comprising carbon dioxide and at least a portion of the methane of the methane synthesis product stream;
contacting the dry methane reformation feed stream with a dry methane reformation catalyst (e.g., in a dry methane reformation reactor) to produce a dry methane reformer product stream comprising hydrogen and carbon monoxide.

2. The process of claim 1, further comprising electrolyzing water to form a hydrogen gas containing stream, and providing at least a portion of the hydrogen gas containing stream to the methane synthesis feed stream, wherein the electrolyzing is performed using electricity from a renewable source.

3. The process of claim 1 or claim 2, wherein the carbon dioxide of the methane synthesis feed stream comprises at least 50%, at least 75%, at least 90%, or at least 95% captured carbon dioxide and/or carbon dioxide from biomass gasification

4. The process of any of claims 1-3 wherein provision of at least a portion of the methane of the methane synthesis product stream to the dry methane reformation feed stream comprises separating at least a portion of the water from the methane of the methane synthesis product stream.

5. The process of any of claims 1-4, wherein the process comprises electrolyzing at least a portion of the separated water to form a hydrogen gas containing stream and an oxygen gas containing stream, and providing at least a portion of the hydrogen gas to the methane synthesis feed stream, the process further comprising providing at least a portion of the separated water as a feed to the electrolyzing.

6. The process of any of claims 1-5, wherein the dry methane reformation feed stream comprises no more than 10 vol% water, e.g., no more than 7.5 vol% water, or no more than 5 vol% water, or no more than 3 vol% water, or no more than 1 vol% water).

7. The process of any of claims 1-6, wherein the methane synthesis product stream comprises carbon dioxide, and wherein at least a portion of the carbon dioxide of the methane synthesis product stream is provided to the dry methane reformation feed.

8. The process of any of claims 1-7, wherein the process further comprises providing at least a portion of the dry methane reformer product stream to the H₂/CO stream (e.g., providing the dry methane reformer product stream as the H₂/CO stream).

9. The process of any of claims 1-7, wherein the process further comprises adjusting the methane reformer product stream to provide an H₂/CO stream containing hydrogen and carbon monoxide with a H₂:CO ratio in the range of 1.5:1 to 3:1.

10. A process for the production of hydrocarbons, the process comprising
providing an H₂/CO stream containing hydrogen and carbon monoxide according to the process of any of claims 1-9;
providing a Fischer-Tropsch feed stream comprising the stream containing hydrogen and carbon monoxide;
contacting the Fischer-Tropsch feed stream with a Fischer-Tropsch catalyst (e.g., in a Fischer-Tropsch reactor) to produce a Fischer-Tropsch product stream comprising C₅₊ hydrocarbons.

11. The process of claim 10, further comprising separating a light hydrocarbon stream from the Fischer-Tropsch product stream, and providing at least a portion of the light hydrocarbon stream to a partial oxidation reactor, and reacting the light hydrocarbon stream with oxygen in the partial oxidation reactor to produce a partial oxidation product stream comprising carbon monoxide and hydrogen.

12. The process of claim 10 or claim 11, wherein the process comprises electrolyzing water to produce a hydrogen gas containing stream and an oxygen gas containing stream, wherein at least a portion of the oxygen gas containing stream is provided to the partial oxidation reactor.

13. The process of any of any of claims 10-12, wherein the partial oxidation product stream comprises carbon dioxide, wherein the process further comprises providing at least a portion of the carbon dioxide of the partial oxidation product stream to at least one of the methane synthesis feed stream and the dry methane reformation feed stream.

14. A process for producing a H₂/CO stream containing hydrogen and carbon monoxide, the processing comprising:
providing a dry methane reformation feed stream comprising biogenic carbon dioxide and biomethane;
contacting the dry methane reformation stream with a dry methane reformation catalyst to produce a dry methane reformer product stream comprising carbon monoxide and hydrogen; and
optionally adjusting the methane reformer product stream to provide a stream containing hydrogen and carbon monoxide with a H₂:CO ratio in the range of 1.5:1 to 3:1.

15. A process for the production of hydrocarbons, the process comprising
providing a H₂/CO stream containing hydrogen and carbon monoxide according to the method of any of claim 14; and
providing a Fischer-Tropsch feed stream comprising the stream containing hydrogen and carbon monoxide;
contacting (e.g., in a Fischer-Tropsch reactor) the Fischer-Tropsch feed stream with a Fischer-Tropsch catalyst to produce a Fischer-Tropsch product stream comprising hydrocarbons, water and optionally oxygenated hydrocarbons.
